# EUROPEAN PATENT APPLICATION

(11) **EP 4 406 501 A1**
(43) Date of publication of application: **31.07.2024**
(21) Application number: 24152787.8
(22) Date of filing: 19.01.2024
(51) Int. Cl.: A61B 18/12, A61B 18/00, A61B 18/14

(54) **RADIOFREQUENCY ABLATION SYSTEM USING NO-TOUCH TECHNIQUE**

(30) Priority: 26.01.2023 KR 20230010147
(71) Applicant: Starmed Co., Ltd., Gyeonggi-do 10442 (KR)
(72) Inventor: SHIN, Kyung Hoon, 10111 Gimpo-si, Gyeonggi-do (KR); LEE, Jeong Min, 06549 Seocho-gu, Seoul (KR); LEE, Min Woo, 06213 Gangnam-gu, Seoul (KR); KIM, Jin Woong, 61704 Nam-gu, Gwangju (KR); LEE, Jun Hyok, 10084 Gimpo-si, Gyeonggi-do (KR); ZU, Jung Hyuk, 01865 Nowon-gu, Seoul (KR); KIM, Dong Un, 10068 Gimpo-si, Gyeonggi-do (KR); PARK, Gye Seong, 10916 Paju-si, Gyeonggi-do (KR)
(74) Representative: Stöckeler, Ferdinand

(57) **Abstract**

Proposed are a radiofrequency ablation system and a method of controlling the radiofrequency ablation system, the system and the method being capable of selectively executing a monopolarity mode, in which heat is concentratedly generated around each of one pair of electrodes, or a bipolarity mode, in which heat is concentratedly generated between one pair of electrodes, without increasing pressure with a lesion by inserting a plurality of electrodes into the surrounding tissue instead of direct insertion thereof into the lesion. With the system and the method, efficiency in heat generation can be maximized, and a tumor recurrence ratio can be reduced.

## Description

### CROSS REFERENCE TO RELATED APPLICATION

The present application claims priority from Korean Patent Application No. 10- 2023-0010147 filed on January 26, 2023, in the Korean Intellectual Property Office, which is incorporated herein by reference in its entirety.

### BACKGROUND OF THE INVENTION

### Field of the Invention

The present disclosure relates to a radiofrequency ablation system using a no-touch technique and a method of controlling the radiofrequency ablation system, and more particularly, to a radiofrequency ablation system using a no-touch technique and a method of controlling the radiofrequency ablation system, the system and the method being capable of selectively executing a monopolarity mode, in which heat is concentratedly generated around each of one pair of electrodes, or a bipolarity mode, in which heat is concentratedly generated between one pair of electrodes, without increasing pressure with a lesion, by inserting a plurality of electrodes into the surrounding tissue instead of direct insertion thereof into the lesion. With the system and the method, efficiency in heat generation can be maximized, and a tumor recurrence ratio can be reduced.

### Background Art

A radiofrequency ablation (RFA) medical treatment technique is a method of performing a medical treatment by raising the temperature of the human tissue to 60°C to 100°C in such a manner that high-frequency energy concentrates on the center portion of a tumor by inserting a needle-shaped electrode into the center portion of the tumor and applying the high-frequency energy. However, in the case of radiofrequency ablation using a single electrode, it is difficult to perform sufficient cauterization on the tumor and the tissue surrounding the tumor, when compared with a surgical treatment. Thus, it has been reported that a tumor recurrence ratio is up to approximately 30% after radiofrequency ablation. Moreover, the frequency of tumor spread is high.

In order to lower the tumor recurrence or the occurrence of tumor spread after radiofrequency ablation, there is a need to create a wider cauterization range per unit hour. Accordingly, multi-electrode radiofrequency ablation has been attempted to increase efficiency in radiofrequency ablation. However, due to an increase in the distance between electrodes, a problem arises in that there occurs a likelihood that cauterization will be incompletely performed on the middle portion between the electrodes.

### Description of the Related Art

### Document of Related Art

(Patent Document 1) Korean Patent No. 2004-0074541 (published on August 25, 2004)

### SUMMARY OF THE INVENTION

An object of the present disclosure is to provide a radiofrequency ablation system using a no-touch technique and a method of controlling the radiofrequency ablation system, the system and the method being capable of selectively executing a monopolarity mode, in which heat is concentratedly generated around each of one pair of electrodes, or a bipolarity mode, in which heat is concentratedly generated between one pair of electrodes, without increasing pressure with a lesion, by inserting a plurality of electrodes into the surrounding tissue instead of direct insertion thereof into the lesion. With the system and the method, efficiency in heat generation can be maximized, and a tumor recurrence ratio can be reduced.

The present disclosure is not limited to the above-mentioned object. From the following description, other objects not mentioned would be clearly understandable to a person of ordinary skill in the art to which the present disclosure pertains.

In order to address the above-mentioned object, according to one aspect of the present disclosure, there is provided a radiofrequency ablation system including: a main amplifier configured to amplify electric power supplied to the main amplifier, the main amplifier being configured to provide main radiofrequency (RF) electric power resulting from amplification by the main amplifier; a sub-amplifier configured to amplify electric power supplied to the sub-amplifier, the sub-amplifier being configured to provide sub-RF electric power resulting from amplification by the sub-amplifier; a first switching circuit configured to transfer the main RF electric power provided by the main amplifier to any one of a plurality of electrodes including at least three electrodes; a second switching circuit configured to transfer the sub-RF electric power provided by the sub-amplifier to any one of the plurality of electrodes; a third switching circuit configured to connect a main ground pad or any one of the plurality of electrodes to a main grounding line; and a fourth switching circuit configured to connect a sub-ground pad or any one of the plurality of electrodes to a sub-grounding line, wherein the radiofrequency ablation system is configured to selectively execute a monopolarity mode or a bipolarity mode according to operations of the first, second, third, and fourth switching circuits, wherein, in the monopolarity mode, the radiofrequency ablation system is configured to supply the main RF electric power to one of one predetermined pair of electrodes, among the plurality of electrodes, and the sub-RF electric power to the other of the one predetermined pair of electrodes, and wherein, in the bipolarity mode, the radiofrequency ablation system is configured to supply either the main RF electric power or the sub-RF electric power to one of the one predetermined pair of electrodes, the radiofrequency ablation system being configured to connect either the main grounding line or the sub-grounding line to the other of the one predetermined pair of electrodes.

In the radiofrequency ablation system, the plurality of electrodes may be configured to be inserted into a surrounding tissue to surround a lesion, without direct insertion into the lesion.

In the radiofrequency ablation system, in the monopolarity mode, the main ground pad may be configured to be connected to the main grounding line, and the sub-ground pad may be configured to be connected to the sub-grounding line.

In the radiofrequency ablation system, in the bipolarity mode, when the main RF electric power is supplied to one of the one predetermined pair of electrodes, the main grounding line may be configured to be connected to the other one of the one predetermined pair of electrodes, and, when the sub-RF electric power is supplied to one of the one predetermined pair of electrodes, the sub-grounding line may be configured to be connected to the other of the one predetermined pair of electrodes.

In the radiofrequency ablation system, the one predetermined pair of electrodes that operate in the monopolarity mode or the bipolarity mode may be configured to be switched to another pair of electrodes when a predetermined time elapses or according to an impedance value of a lesion.

The radiofrequency ablation system may further include: a main board sensor configured to monitor voltage and current on a main amplifier side while the main amplifier outputs the main RF electric power; and a sub-sensor board configured to monitor voltage and current on the sub-amplifier side while the sub-amplifier outputs the sub-RF electric power.

The radiofrequency ablation system may further include a controller configured to continuously compute impedance of a lesion using the voltage and the current that are monitored by the main board sensor and the voltage and the current that are monitored by the sub-sensor board, during radiofrequency ablation, the controller being configured to compare the computed impedance with a preset reference value.

In the radiofrequency ablation system, in a case where a predetermined time elapses or in a case where the impedance exceeds the preset reference value, the controller may be configured to control the first, second, third, and fourth switching circuits to switch the one predetermined pair of electrodes that operate in the monopolarity mode or the bipolarity mode to another pair of electrodes.

In the radiofrequency ablation system, the radiofrequency ablation system may be configured to selectively execute one of the monopolarity mode, the bipolarity mode, and a hybrid mode, and, in the hybrid mode, the radiofrequency ablation system may be configured to supply either the main RF electric power or the sub-RF electric power to one of the one predetermined pair of electrodes, and the other of the one predetermined pair of electrodes and the main ground pad may be configured to be simultaneously connected to the main grounding line, or the other of the one predetermined pair of electrodes and the sub-ground pad may be configured to be simultaneously connected to the sub-grounding line.

In radiofrequency ablation system, the one predetermined pair of electrodes that operate in the hybrid mode may be configured to be switched to another pair of electrodes when the predetermined time elapses or according to the impedance value of the lesion.

In the radiofrequency ablation system, in a case where the plurality of electrodes includes first, second, and third electrodes, the first switching circuit may include: a first switch configured to transfer the main RF electric power to the first electrode; a second switch configured to transfer the main RF electric power to the second electrode; and a third switch configured to transfer the main RF electric power to the third electrode, and the second switching circuit may include: an eighth switch configured to transfer the sub-RF electric power to the first electrode; a ninth switch configured to transfer the sub-RF electric power to the second electrode; and a tenth switch configured to transfer the sub-RF electric power to the third electrode.

In the radiofrequency ablation system, the third switching circuit may include: a fourth switch configured to connect the main ground pad to the main grounding line; a fifth switch configured to connect the first electrode to the main grounding line; a sixth switch configured to connect the second electrode to the main grounding line; and a seventh switch configured to connect the third electrode to the main grounding line, and the fourth switching circuit may include: eleventh switch configured to connect the sub-ground pad to the sub-grounding line; a twelfth switch configured to connect the first electrode to the sub-grounding line; a thirteenth switch configured to connect the second electrode to the sub-grounding line; and a fourteenth switch configured to connect the third electrode to the sub-grounding line.

In order to address the above-mentioned object, according to another aspect of the present disclosure, there is provided a method of controlling radiofrequency ablation system that uses a plurality of electrodes having at least three electrodes, the method comprising: selecting either monopolarity mode or bipolarity mode; performing switching in a manner that main radiofrequency (RF) electric power is supplied to one of one predetermined pair of electrodes, among the plurality of electrodes, and sub-RF electric power is supplied to the other of the one predetermined pair of electrodes, when selecting the monopolarity mode, and in a manner that either the main RF electric power or the sub-RF electric power is supplied to one of the one predetermined pair of electrodes, and either main grounding line or a sub-grounding line is connected to the other of the predetermined pair of electrodes, when selecting the bipolarity mode; monitoring voltage and current on a main amplifier side while a main amplifier outputs the main RF electric power, and monitoring voltage and current on a sub-amplifier side while a sub-amplifier outputs the sub-RF electric power; computing continuously impedance of a lesion using the voltage and the current on the main amplifier side and the voltage and the current on the sub-amplifier side during radiofrequency ablation and comparing the impedance with a preset reference value; and performing another switching, wherein the one predetermined pair of electrodes that operate in the monopolarity mode or the bipolarity mode is switched to another pair of electrodes, in a case where a predetermined time elapses or where the impedance exceeds the preset reference value.

The method may further include inserting the plurality of electrodes in a surrounding tissue to surround a lesion, without direct insertion into the lesion on which cauterization is to be performed.

In the method, the selecting is performed by selecting one of the monopolarity mode, the bipolarity mode, and a hybrid mode; when the hybrid mode is selected, switching in the hybrid mode may be performed in a manner that either the main RF electric power or the sub-RF electric power is supplied to one of the one predetermined pair of electrodes and in a manner that the other of the one predetermined pair of electrodes and a main ground pad are simultaneously connected to the main grounding line by the switching in the hybrid mode, or that the other of the one predetermined pair of electrodes and a sub-ground pad are simultaneously connected to the sub-grounding line by the switching in the hybrid mode; and another switching in the hybrid mode may be performed, wherein the one predetermined pair of electrodes that operate in the hybrid mode is switched to another pair of electrodes, in the case where the predetermined time elapses or where the impedance exceeds the preset reference value.

According to the present disclosure, cauterization can be performed without increasing pressure within the lesion by inserting the plurality of electrodes into the surrounding tissue without direct insertion thereof into the lesion.

In addition, the radiofrequency ablation system according to the present disclosure is capable of selectively executing a monopolarity mode, in which heat is concentratedly generated around each of one pair of electrodes, or a bipolarity mode, in which heat is concentratedly generated between one pair of electrodes. Thus, the efficiency in heat generation can be maximized, and the tumor recurrence ratio can be reduced. That is, with a wide cauterization range, it is possible to perform complete cauterization on the lesion without direct insertion of the plurality of electrodes into the lesion.

In addition, if the hybrid mode is selected, impedance of the lesion can be decreased by simultaneously connecting the grounding line to the ground pad and one of one pair of electrodes.

The present disclosure is not limited to the above-mentioned effects. It should be understood from the detailed description of the present disclosure or the limitations of the claims that other effects can be deducted from the detailed description of the present disclosure or the limitations of the claims.

### BRIEF DESCRIPTION OF THE DRAWINGS

FIG. 1 is a schematic diagram illustrating a radiofrequency ablation system according to a first embodiment.
FIG. 2 is a perspective view illustrating a state where a plurality of electrodes is inserted into the tissue surrounding a lesion.
FIGS. 3A, 4A, and 5A are schematic diagrams illustrating first to third states, respectively, in a monopolarity mode in FIG. 1.
FIGS. 3B, 4B, and 5B are front diagrams illustrating cauterization ranges, respectively, of the plurality of electrodes in the states illustrated in FIGS. 3A, 4A, and 5A.
FIGS. 6A, 7A, and 8A are schematic diagrams illustrating first to third states, respectively, in a bipolarity mode in FIG. 1.
FIGS. 6B, 7B, and 8B are front diagrams illustrating cauterization ranges, respectively, of the plurality of electrodes in the states illustrated in FIGS. 6A, 7A, and 8A.
FIGS. 9 to 11 are schematic diagrams illustrating first to third states, respectively, in a hybrid mode in FIG. 1.
FIG. 12 is a flowchart illustrating a method of controlling the radiofrequency ablation system according to a second embodiment.

### DETAILED DESCRIPTION OF THE INVENTION

A radiofrequency ablation system and a method of controlling the radiofrequency ablation system according to preferred embodiments of the present disclosure will be described below with reference to the accompanying drawings.

In addition, terms defined by considering the meanings thereof in the present disclosure will be used below and may vary according to the user's or manager's intention or according to practices in the art. The preferred embodiments described below, which each contain limitations recited in the claims of the present disclosure, are provided only for illustrative purposes without imposing limitations on the scope of the present disclosure.

To provide a concise description of the preferred embodiments of the present disclosure, non-relevant content is omitted. Throughout the specification, the same reference numerals are assigned to identical or similar constituent elements. When the expression "includes a constituent element" is used throughout the specification, unless otherwise particularly described, this expression means "further includes any other constituent element," not "excludes any other constituent element."

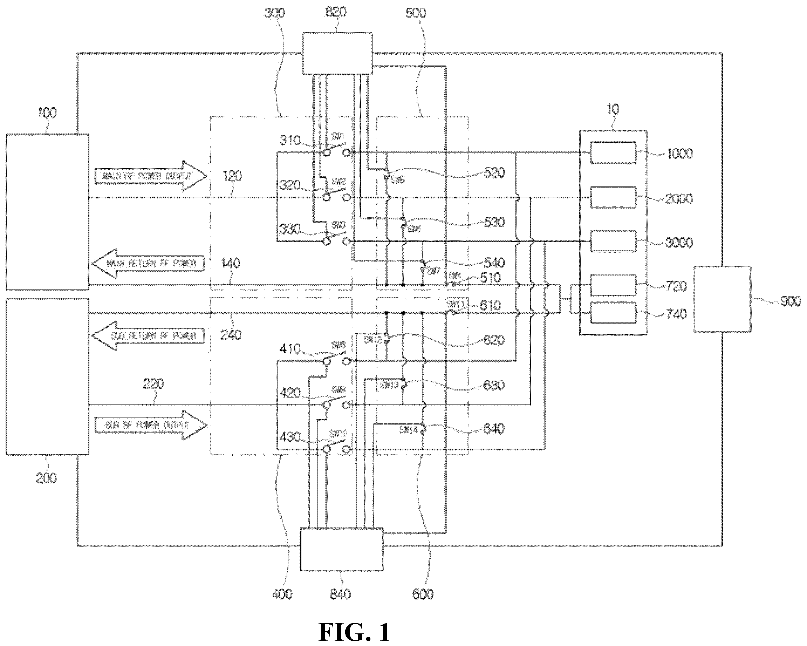

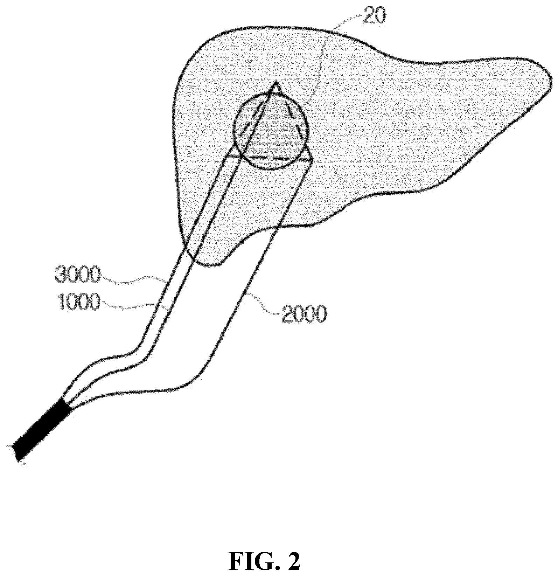

First, a radiofrequency ablation system according to a first embodiment of the present disclosure is described with reference to FIGS. 1 and 2.

The radiofrequency ablation system according to the first embodiment of the present disclosure may broadly include a main amplification unit 100, a sub-amplification unit 200, a first switching unit 300, a second switching unit 400, a third switching unit 500, a fourth switching unit 600, a main ground pad 720, a sub-ground pad 740, and a plurality of electrodes. In the present embodiment, the plurality of electrodes are described as including first, second, and third electrodes 1000, 2000, and 3000, but are not limited thereto.

The main amplification unit 100 amplifies electric power supplied by a main switching mode power supply (SMPS) and provides main RF electric power resulting from the amplification. A main output line 120 over which the main RF electric power is output and a main grounding line 140 over which the main RF electric power is returned are connected to the main amplification unit 100. The main output line 120 corresponds to being active (+), and the main grounding line 140 corresponds to being passive (-).

The sub-amplification unit 200 amplifies electric power supplied by a sub-SMPS and provides sub-RF electric power resulting from the amplification. A sub-output line 220 over which the sub-RF electric power is output and a sub-grounding line 240 over which the sub-RF electric power is returned are connected to the sub-amplification unit 200. The sub-output line 220 corresponds to being active (+), and the sub-grounding line 240 corresponds to being passive (-).

The first switching unit 300 transfers the main RF electric power provided by the main amplification unit 100 to any one of the first, second, and third electrodes 1000, 2000, and 3000. To this end, the first switching unit 300 may provide the main RF electric power over the main output line 120. Specifically, the first switching unit 300 includes a first switch (SW1) 310 for transferring the main RF electric power to the first electrode 1000, a second switch (SW2) 320 for transferring the main RF electric power to the second electrode 2000, and a third switch (SW3) 330 for transferring the main RF electric power to the third electrode 3000.

The second switching unit 400 transfers the sub-RF electric power provided by the sub-amplification unit 200 to any one of the first, second, and third electrodes 1000, 2000, and 3000. To the end, the second switching unit 400 may provide the sub-RF electric power over the sub-output line 220. Specifically, the second switching unit 400 includes an eighth switch (SW8) 410 for transferring the sub-RF electric power to the first electrode 1000, a ninth switch (SW9) 420 for transferring the sub-RF electric power to the second electrode 2000, and the tenth switch (SW10) 430 for transferring the sub-RF electric power to the third electrode 3000.

The third switching unit 500 connects the main ground pad 720 or any one of the first, second, and third electrodes 1000, 2000, and 3000 to the main grounding line 140. Specifically, the third switching unit 500 includes a fourth switch (SW4) 510 for connecting the main ground pad 720 to the main grounding line 140, a fifth switch (SW5) 520 for connecting the first electrode 1000 to the main grounding line 140, a sixth switch (SW6) 530 for connecting the second electrode 2000 to the main grounding line 140, and a seventh switch (SW7) 540 for connecting the third electrode 3000 to the main grounding line 140.

At this point, the fifth switch 520, the sixth switch 530, and the seventh switch 540 are connected to rear ends, respectively, of the first switch 310, the second switch 320, and the third switch 330, but are connected more forward than the fourth switch 510.

The fourth switching unit 600 connects the sub-ground pad 740 or any one of the first, second, and third electrodes 1000, 2000, and 3000 to the sub-grounding line 240. Specifically, the fourth switching unit 600 includes an eleventh switch (SW11) 610 for connecting the sub-ground pad 740 to the sub-grounding line 240, the twelfth switch (SW12) 620 for connecting the first electrode 1000 to the sub-grounding line 240, a thirteenth switch (SW13) 630 for connecting the second electrode 2000 to the sub-grounding line 240, and a fourteenth switch (SW14) 640 for connecting the third electrode 3000 to the sub-grounding line 240.

At this point, the twelfth switch 620, the thirteenth switch 630, and the fourteenth switch 640 are connected to rear ends, respectively, of the eighth switch 410, the ninth switch 420, and the tenth switch 430, but are connected more forward than the eleventh switch 610.

The first, second, third, and fourth switching units 300, 400, 500, and 600 may be on (closed) or off (open) according to control signals of a control unit 900 described below.

At this point, the main ground pad 720 and the sub-ground pad 740 may be attached to the body of a patient 10.

In the radiofrequency ablation system according to the present disclosure, a monopolarity mode or a bipolarity mode is selectively executable according to operations of the first, second, third, and fourth switching units 300, 400, 500, and 600. In the monopolarity mode, the main RF electric power and the sub-RF electric power are supplied to one predetermined pair of electrodes, respectively, among the first, second, and third electrodes 1000, 2000, and 3000. In the bipolarity mode, one of the following: the main RF electric power; and the sub-RF electric power is supplied to one of the one predetermined pair of electrodes, and one of the main grounding and sub-grounding lines 140 and 240 is connected to the other one.

Accordingly, in the monopolarity mode, heat can be concentratedly generated around each of one pair of electrodes, and in the bipolarity mode, heat can be generated between a pair of electrodes. Thus, efficiency in heat generation can be maximized, and a tumor recurrence ratio can be reduced. That is, a cauterization range can be widened, when compared with the case where only the monopolarity mode is executed.

Particularly, as illustrated in FIG. 2, no-touch technique in which an electrode does not make direct contact with a lesion can be used. Accordingly, the first, second, and third electrodes 1000, 2000, and 3000 can be inserted into the surrounding tissue in such a manner as to surround a lesion 20, without direct insertion thereof into the lesion 20 on which cauterization is required to be performed. As an example, the first, second, and third electrodes 1000, 2000, and 3000 may be arranged, in the shape of a triangle, around the lesion 20. Accordingly, cauterization can be performed without increasing pressure within the lesion 20, by inserting a plurality of electrodes in the surrounding tissue instead of direct insertion thereof into the lesion 20. In addition, as described above, the radiofrequency ablation system according to the present disclosure is operable in both the monopolarity mode and the bipolarity mode. Thus, even though a plurality of electrodes are not directly inserted into the lesion 20, since the cauterization range is wide, complete cauterization of the lesion 20 is possible.

At this point, one pair of electrodes that operate in the monopolarity mode or the bipolarity mode may be switched to another pair of electrodes when a predetermined time elapses or according to an impedance value of the lesion 20. As an example, one pair of electrodes that operate in the monopolarity mode or the bipolarity mode may be relay-controlled to be switched to a pair of the first and second electrodes 1000 and 2000, a pair of the second and third electrodes 2000 and 3000, and a pair of the third and first electrodes 3000 and 1000 in this order. Information on these pairs of electrodes may be pre-stored in the control unit 900.

To this end, the radiofrequency ablation system according to the present disclosure may further include a main board sensor 820, a sub-sensor board 840, and the control unit 900.

The main board sensor 820 monitors voltage and current on the main amplification unit 100 side while the main amplification unit 100 outputs the main RF electric power. The sub-sensor board 840 monitors voltage and current on the sub-amplification unit 200 side while the sub-amplification unit 200 outputs the sub-RF electric power. Specifically, during radiofrequency ablation, the main board sensor 820 and the sub-sensor board 840 read the high-frequency root-mean-square current Irms flowing through the tissue and the high-frequency root-mean-square voltage Vrms applied to both ends of the tissue and transfer this information to the control unit 900. Thus, the control unit 900 can compute the impedance.

In a case where the main board sensor 820 is implemented in such a manner as to have an A/D conversion function and where the sub-sensor board 840 is implemented in such a manner as not to have an A/D conversion function, the sub-sensor board 840 may transfer values of the high-frequency root-mean-square current and voltage to the main board sensor 820. The values resulting from A/D conversion by the main board sensor 820 may be transferred to the control unit 900.

During the radiofrequency ablation, the control unit 900 continuously computes the impedance of the lesion 20 using the monitored voltage and current and compares the computed impedance with a preset reference value.

Accordingly, in a case where the predetermined time elapses or where the computed impedance exceeds the preset reference value, the control unit 900 controls the first, second, third, and fourth switching units 300, 400, 500, and 600 in such a manner that one pair of electrodes that operate in the monopolarity mode or the bipolarity mode is switched to another pair of electrodes. This specific process is in detail described.

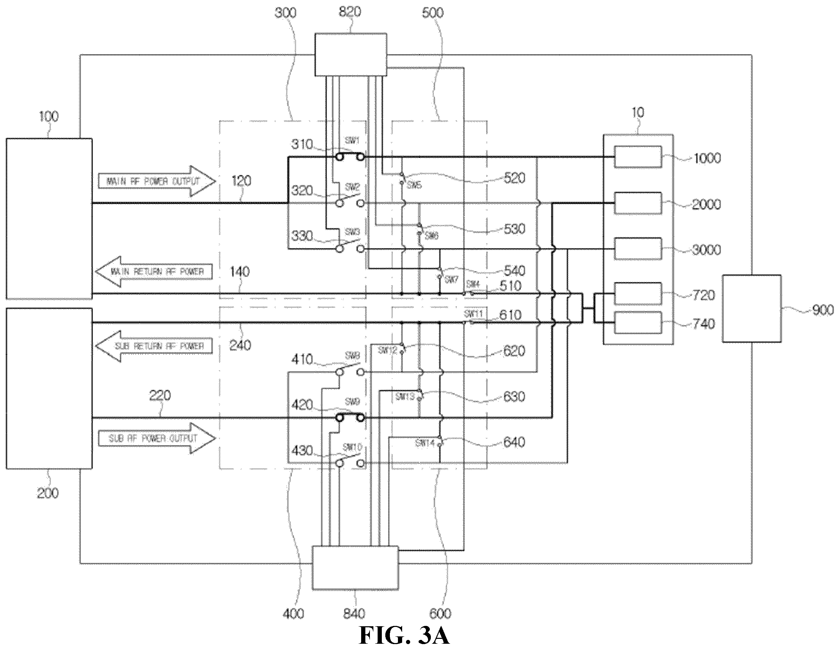

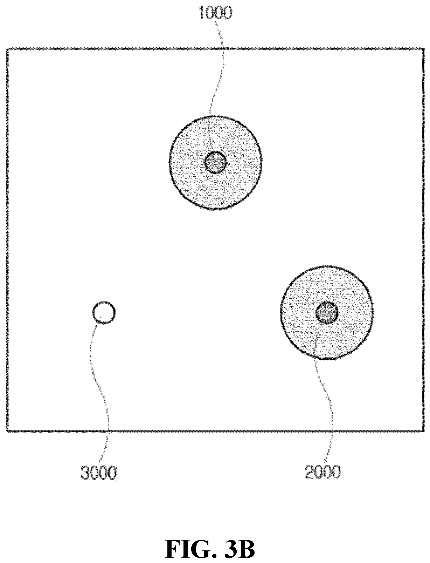

The operation in the monopolarity mode is described in detail below with reference to FIGS. 3 to 5.

In the monopolarity mode, the main RF electric power and the sub-RF electric power are supplied to one predetermined pair of electrodes, respectively, among the first, second, and third electrodes 1000, 2000, and 3000. That is, when any one of the first, second, and third switches 310, 320, and 330 is closed (on), the main RF electric power is supplied to any one of the first, second, and third electrodes 1000, 2000, and 3000. Furthermore, when any one of the eighth, ninth, and tenth switches 410, 420, and 430 is closed, the sub-RF electric power is supplied to any one of electrodes to which the main RF electric power is not supplied, among the first, second, and third electrodes 1000, 2000, and 3000.

As described above, one pair of electrodes that operate is relay-controlled to be switched to the pair of the first and second electrodes 1000 and 2000, the pair of the second and third electrodes 2000 and 3000, and the pair of the third and first electrodes 3000 and 1000. As a result, as illustrated in FIG. 3A, when the monopolarity mode is executed, the first switch 310 is closed, and thus the main RF electric power can be supplied to the first electrode 1000. Furthermore, the ninth switch 420 is closed and thus, the sub-RF electric power can be supplied to the second electrode 2000. At this point, the fourth switch 510 is closed, and thus, the main ground pad 720 is connected to the main grounding line 140. Furthermore, the eleventh switch 610 is closed, and thus the sub-ground pad 740 is connected to the sub-grounding line 240.

Accordingly, the main RF electric power, output to the first electrode 1000, is turned to the main amplification unit 100 through the main ground pad 720, and the sub-RF electric power, output to the second electrode 2000, is returned to the sub-amplification unit 200 through the sub-ground pad 740.

FIG. 3B illustrates a cauterization range of each of the first, second, and third electrodes 1000, 2000, and 3000. From FIG. 3B, it can be seen that, since in the monopolarity mode, heat is concentratedly generated around each of the first electrode 1000 and the second electrode 2000 that operate, cauterization ranges in the form of a circle are formed around the first electrode 1000 and the second electrode 2000, respectively. The third electrode 3000 is in an off state.

In this state, in a case where the control unit 900 determines that the predetermined time elapses or that the computed impedance exceeds the preset reference value, the control unit 900 controls the first, second, third, and fourth switching units 300, 400, 500, and 600 in such a manner that the pair of the first and second electrodes 1000 and 2000 that operate is switched to another pair of electrodes, that is, the second and third electrodes 2000 and 3000.

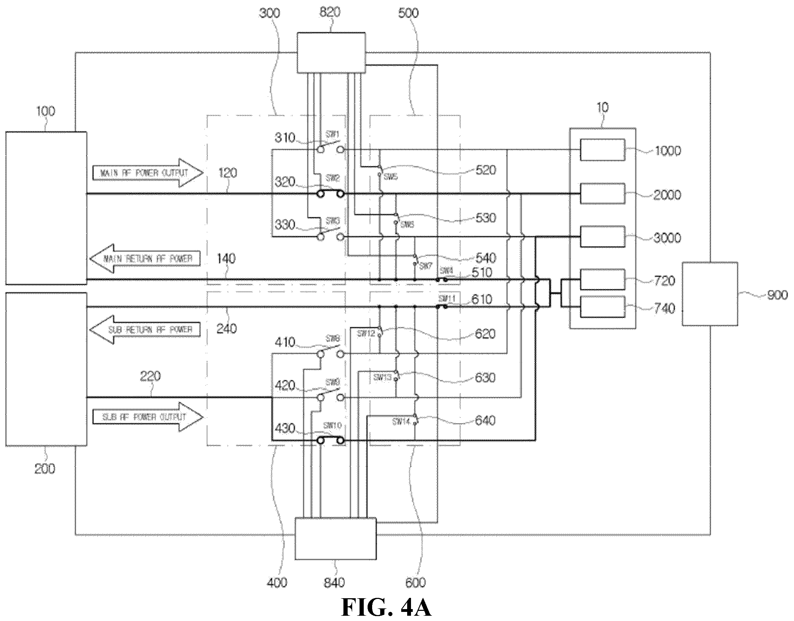

Specifically, as illustrated in FIG. 4A, the first switch 310 is open (off) and the second switch 320 is closed (on) in such a manner that the main RF electric power is supplied to the second electrode 2000. Furthermore, the ninth switch 420 is open and the tenth switch 430 is closed in such a manner that the sub-RF electric power is supplied to the third electrode 3000. At this time, the fourth switch 510 and the eleventh switch 610 are kept in a closed state.

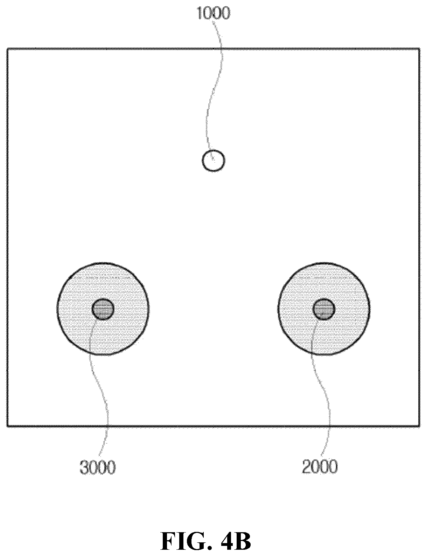

Accordingly, the main RF electric power, output to the second electrode 2000, is turned to the main amplification unit 100 through the main ground pad 720, and the sub-RF electric power, output to the third electrode 3000, is returned to the sub-amplification unit 200 through the sub-ground pad 740. From FIG. 4B, it can be seen that cauterization ranges in the form of a circle are formed around the second electrode 2000 and the third electrode 3000, respectively. The first electrode 1000 is in the off state.

Likewise, in this state, in the case where the control unit 900 determines that the predetermined time elapses or that the computed impedance exceeds the preset reference value, the control unit 900 controls the first, second, third, and fourth switching units 300, 400, 500, and 600 in such a manner that the pair of the second and third electrodes 2000 and 3000 that operate is switched to the pair of the third and first electrodes 3000 and 1000.

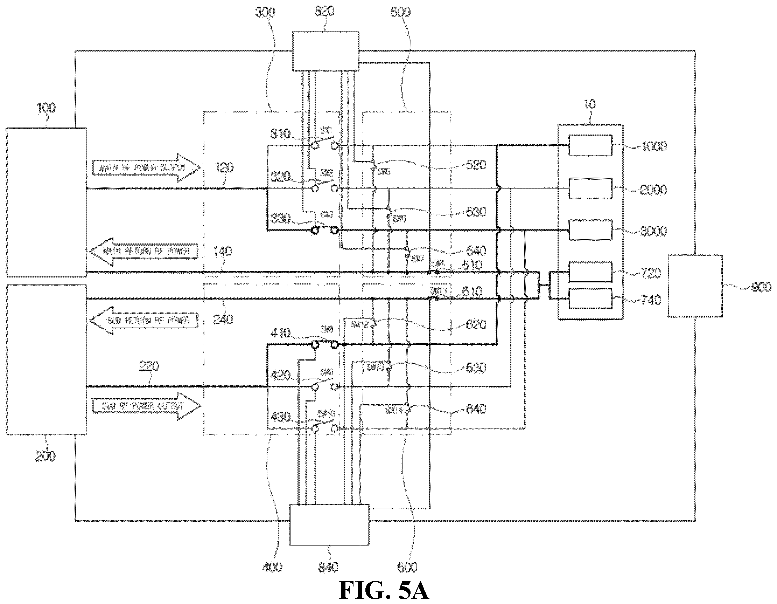

Specifically, as illustrated in FIG. 5A, the second switch 320 is open and the third switch 330 is closed in such a manner that the main RF electric power is supplied to the third electrode 3000. Furthermore, the tenth switch 430 is open and the eighth switch 410 is closed in such a manner that the sub-RF electric power is supplied to the first electrode 1000. At this point, the fourth switch 510 and the eleventh switch 610 are kept in the closed state.

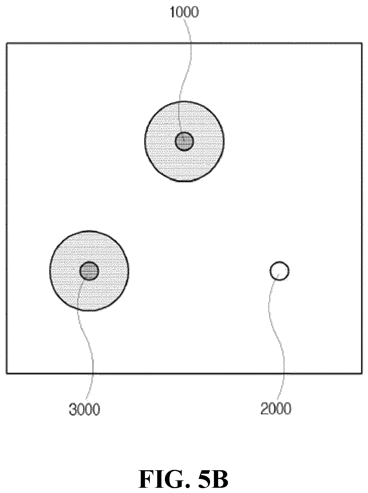

Accordingly, the main RF electric power, output to the third electrode 3000, is turned to the main amplification unit 100 through the main ground pad 720, and the sub-RF electric power, output to the first electrode 1000, is returned to the sub-amplification unit 200 through the sub-ground pad 740. From FIG. 5B, it can be seen that cauterization ranges in the form of a circle are formed around the third electrode 3000 and the first electrode 1000, respectively. The second electrode 2000 is in the off state.

Next, in this state, in the case where the control unit 900 determines that the predetermined time elapses or that the computed impedance exceeds the preset reference value, the control unit 900 may finish the radiofrequency ablation or may control the first, second, third, and fourth switching units 300, 400, 500, and 600 in such a manner that the pair of the third and first electrodes 3000 and 1000 is switched back to the pair of the first and second electrodes 1000 and 2000. Alternatively, the mode may be again selected.

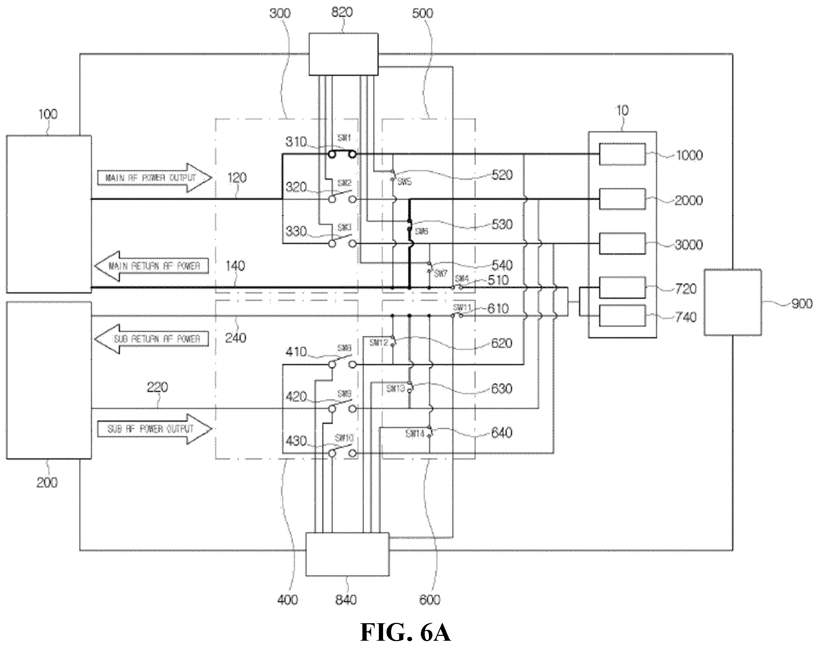

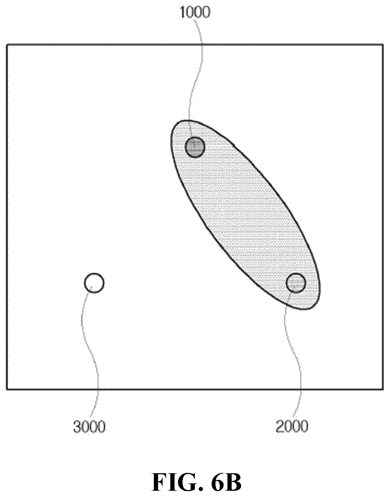

Next, operation in the bipolarity mode is described in detail below with reference to FIGS. 6 to 8.

In the bipolarity mode, one of the following: the main RF electric power; and the sub-RF electric power is supplied to one of the one predetermined pair of electrodes, among the first, second, and third electrodes 1000, 2000, and 3000, and the main grounding line 140 or the sub-grounding line 240 is connected to the other one. That is, when any one of the first, second, and third switches 310, 320, and 330 is closed and thus, the main RF electric power is supplied to any one of the first, second, and third electrodes 1000, 2000, and 3000, any one of electrodes to which the main RF electric power is not supplied, among the first, second, and third electrodes 1000, 2000, and 3000 is connected to the main grounding line 140. Alternatively, when any one of the eighth, ninth, and tenth switches 410, 420, and 430 is closed and thus, the sub-RF electric power is supplied to any one of the first, second, and third electrodes 1000, 2000, and 3000, any one of the remaining electrodes to which the sub-RF electric power is not supplied, among the first, second, and third electrodes 1000, 2000, and 3000 is connected to the sub-grounding line 240.

The following description is provided on the assumption that only the main RF electric power is supplied in the bipolarity mode. However, the present disclosure is not limited thereto and, of course, only the sub-RF electric power may be supplied.

As described above, one pair of electrodes that operate is relay-controlled to be switched to the pair of the first and second electrodes 1000 and 2000, the pair of the second and third electrodes 2000 and 3000, and the pair of the third and first electrodes 3000 and 1000. As a result, as illustrated in FIG. 6A, when the bipolarity mode is executed, the first switch 310 is closed and thus, the main RF electric power is supplied to the first electrode 1000. Furthermore, the sixth switch 530 is closed and thus, the second electrode 2000 is connected to the main grounding line 140.

Accordingly, the main RF electric power, output to the first electrode 1000, is returned to the main amplification unit 100 through the second electrode 2000. FIG. 6B illustrates the first, second, and third electrodes 1000, 2000, and 3000. From 6B, it can be seen that since in the bipolarity mode, heat is concentratedly generated between the first electrode 1000 and the second electrode 2000 that operate, a cauterization range in the form of an ellipse is formed between the first electrode 1000 and the second electrode 2000. The third electrode 3000 is in the off state.

In this state, in the case where the control unit 900 determines that the predetermined time elapses or that the computed impedance exceeds the preset reference value, the control unit 900 controls the first, second, third, and fourth switching units 300, 400, 500, and 600 in such a manner that the pair of the first and second electrodes 1000 and 2000 that operate is switched to another pair of electrodes, that is, the second and third electrodes 2000 and 3000.

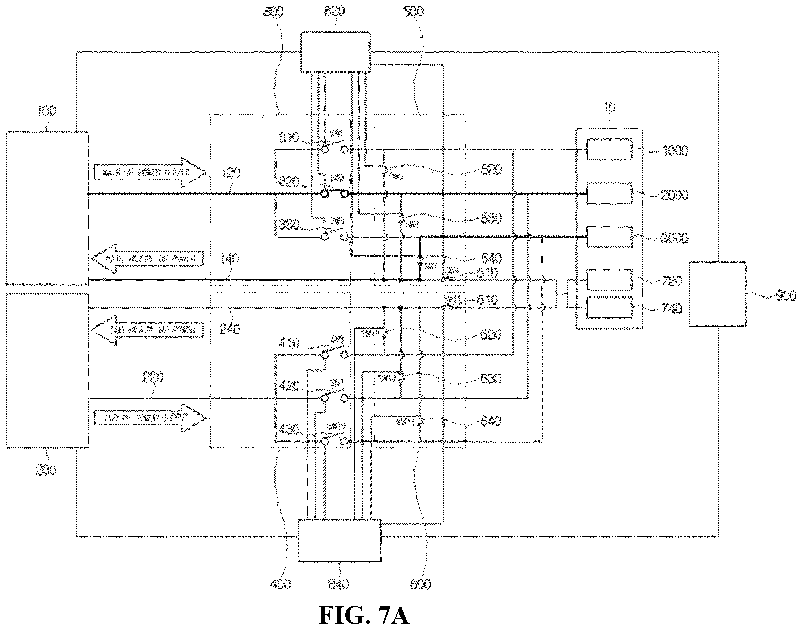

Specifically, as illustrated in FIG. 7A, the first switch 310 is open and the second switch 320 is closed in such a manner that the main RF electric power is supplied to the second electrode 2000. Furthermore, the sixth switch 530 is open and the seventh switch 540 is closed in such a manner that the third electrode 3000 is connected to the main grounding line 140.

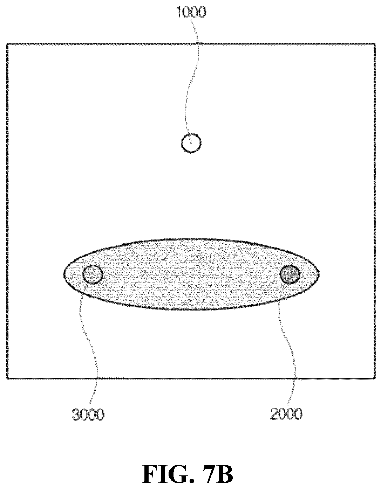

Accordingly, the main RF electric power, output to the second electrode 2000, is returned to the main amplification unit 100 through the third electrode 3000. From FIG. 7B, it can be seen that a cauterization range in the form of an ellipse is formed around the second electrode 2000 and the third electrode 3000. The first electrode 1000 is in the off state.

Likewise, in this state, in the case where the control unit 900 determines that the predetermined time elapses or that the computed impedance exceeds the preset reference value, the control unit 900 controls the first, second, third, and fourth switching units 300, 400, 500, and 600 in such a manner that the pair of the second and third electrodes 2000 and 3000 that operate is switched to the pair of the third and first electrodes 3000 and 1000.

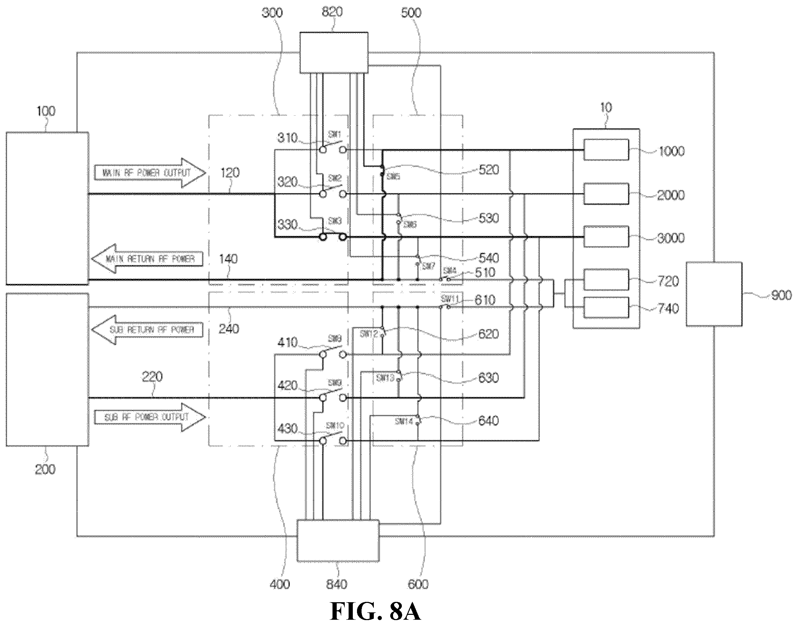

Specifically, as illustrated in FIG. 8A, the second switch 320 is open and the third switch 330 is closed in such a manner that the main RF electric power is supplied to the third electrode 3000. Furthermore, the seventh switch 540 is open and the fifth switch 520 is closed in such a manner that the first electrode 1000 is connected to the main grounding line 140.

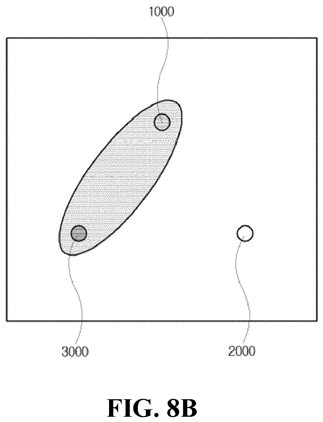

Accordingly, the main RF electric power, output to the third electrode 3000, is returned to the main amplification unit 100 through the first electrode 1000. From FIG. 8B, it can be seen that a cauterization range in the form of an ellipse is formed between the third electrode 3000 and the first electrode 1000. The second electrode 2000 is in the off state.

Next, in this state, in the case where the control unit 900 determines that the predetermined time elapses or that the computed impedance exceeds the preset reference value, the control unit 900 may finish the radiofrequency ablation or may control the first, second, third, and fourth switching units 300, 400, 500, and 600 in such a manner that the pair of the third and first electrodes 3000 and 1000 is switched back to the pair of the first and second electrodes 1000 and 2000. Alternatively, the mode may be again selected.

As an implementation example, in the radiofrequency ablation system according to the present disclosure, a hybrid mode may be selectively executed.

In the hybrid mode, one of the following: the main RF electric power; and the sub-RF electric power is supplied to one of one predetermined pair of electrodes, and the other one of the one predetermined pair of electrodes and the main ground pad 720 are simultaneously connected to the main grounding line 140, or the other one of the one predetermined pair of electrodes and the sub-ground pad 740 are simultaneously connected to the sub-grounding line 240. That is, the hybrid mode may be referred to as a state where in the bipolarity mode, the ground pad is additionally connected to the grounding line.

In this manner, in the hybrid mode, the grounding line is simultaneously connected to the hybrid mode, the ground pad and the electrode are simultaneously connected, and thus the impedance of the lesion 20 can be further decreased.

Likewise, in the hybrid mode, one pair of electrodes that operate may also be switched to another pair of electrodes when the predetermined time elapses or according to the impedance value of the lesion 20. That is, one pair of electrodes that operate in the hybrid mode may be relay-controlled to be switched to the pair of the first and second electrodes 1000 and 2000, the pair of the second and third electrodes 2000 and 3000, and the pair of the third and first electrodes 3000 and 1000 in this order.

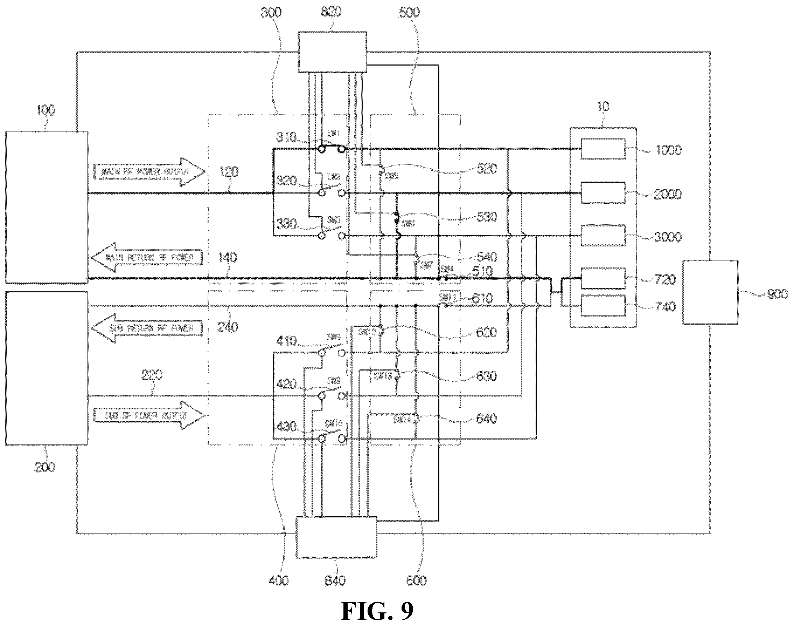

Lastly, operation in the hybrid mode is described in detail with reference to FIGS. 9 to 11.

As illustrated in FIG. 9, when the hybrid mode is executed, the first switch 310 is closed and thus, the main RF electric power is supplied to the first electrode 1000. Furthermore, the fourth switch 510 and the sixth switch 530 are closed and thus, the main ground pad 720 and the second electrode 2000 are simultaneously connected to the main grounding line 140.

Accordingly, the main RF electric power, output to the first electrode 1000, is returned to the main amplification unit 100 through the main ground pad 720 and the second electrode 2000. At this time, likewise, as illustrated in FIG. 6B, a cauterization range in the form of an ellipse is formed between the first electrode 1000 and the second electrode 2000, and the impedance is more decreased than in the bipolarity mode.

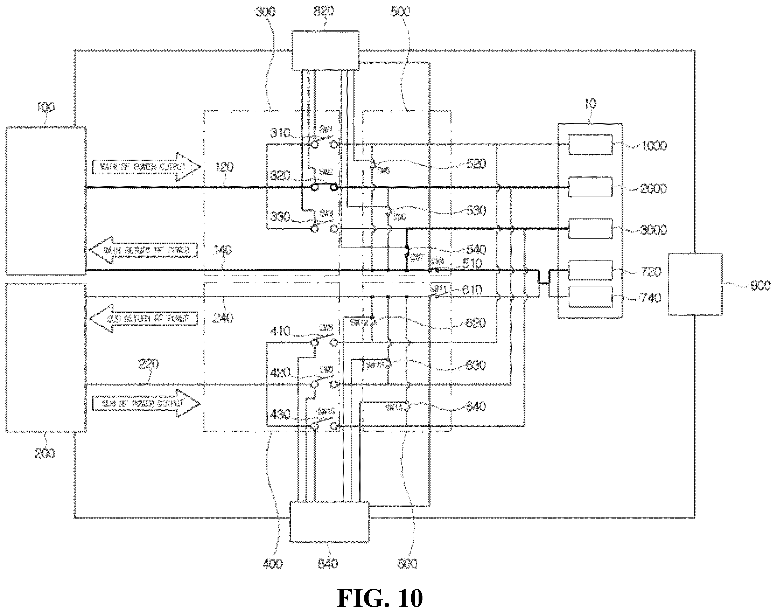

In this state, in the case where the control unit 900 determines that the predetermined time elapses or that the computed impedance exceeds the preset reference value, as illustrated in FIG. 10, the control unit 900 opens the first switch 310 and closes the second switch 320 in such a manner that the main RF electric power is supplied to the second electrode 2000. Furthermore, the control unit 900 opens the sixth switch 530 and closes the seventh switch 540 while keeping the fourth switch 510 closed, in such a manner that the main ground pad 720 and the third electrode 3000 are connected to the main grounding line 140.

Accordingly, the main RF electric power, output to the second electrode 2000, is returned to the main amplification unit 100 through the main ground pad 720 and the third electrode 3000. At this time, likewise, as illustrated in FIG. 7B, a cauterization range in the form of an ellipse is formed between the second electrode 2000 and the third electrode 3000, and the impedance is more decreased than in the bipolarity mode.

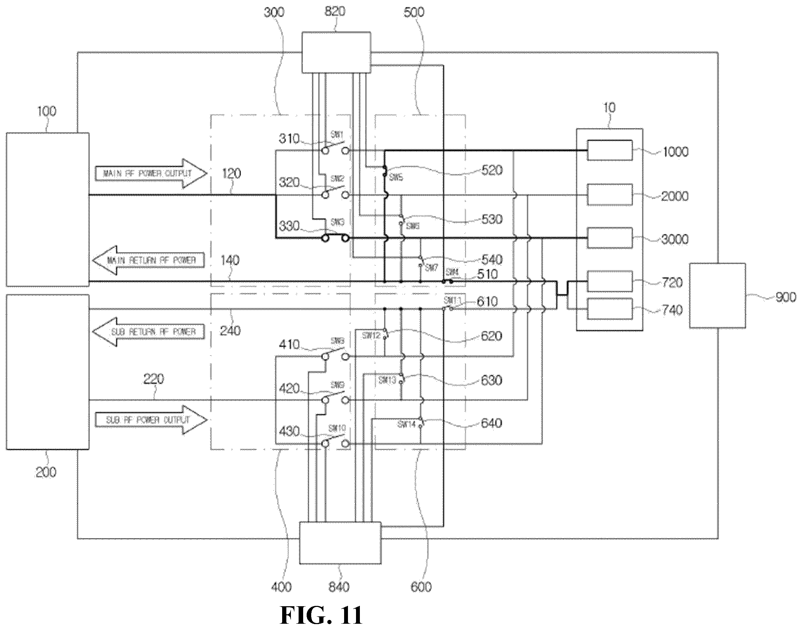

Likewise, in this state, in the case where the control unit 900 determines that the predetermined time elapses or that the computed impedance exceeds the preset reference value, as illustrated in FIG. 11, the control unit 900 opens the second switch 320 and closes the third switch 330 in such a manner that the main RF electric power is supplied to the third electrode 3000. Furthermore, the control unit 900 opens the seventh switch 540 and closes the fifth switch 520 while keeping the fourth switch 510 closed, in such a manner that the main ground pad 720 and the first electrode 1000 are connected to the main grounding line 140.

Accordingly, the main RF electric power, output to the third electrode 3000, is returned to the main amplification unit 100 through the main ground pad 720 and the first electrode 1000. At this time, likewise, as illustrated in FIG. 8B, a cauterization range in the form of an ellipse is formed between the third electrode 3000 and the first electrode 1000, and the impedance is more decreased than in the bipolarity mode.

Next, in this state, in the case where the control unit 900 determines that the predetermined time elapses or that the computed impedance exceeds the preset reference value, the control unit 900 may finish the radiofrequency ablation or may control the first, second, third, and fourth switching units 300, 400, 500, and 600 in such a manner that the pair of the third and first electrodes 3000 and 1000 is switched back to the pair of the first and second electrodes 1000 and 2000. Alternatively, the mode may be again selected.

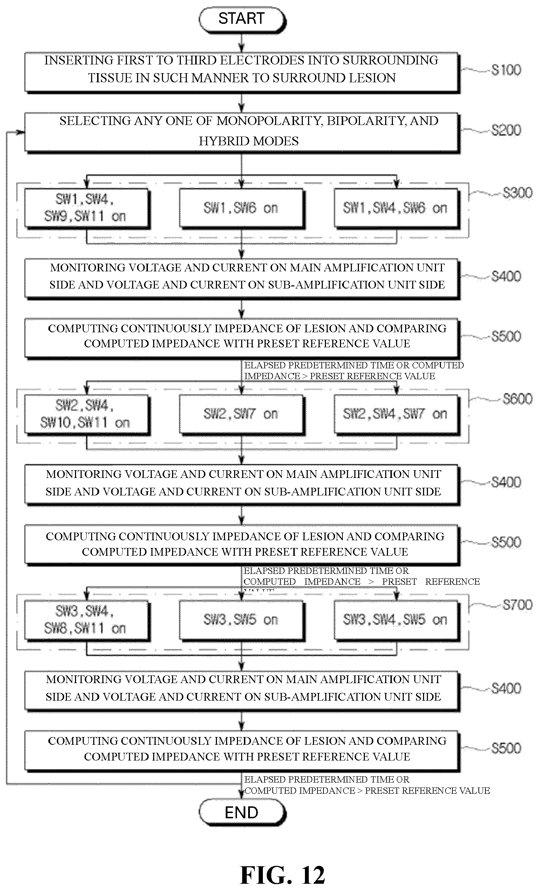

The method of controlling the radiofrequency ablation according to the second embodiment of the present disclosure will be described below with reference to FIG. 12.

First, in the present embodiment, Step (an electrode insertion step) S 100 of inserting a plurality of electrodes, the first, second, and third electrodes 1000, 2000, and 3000 into the surrounding tissue in such a manner as to surround the lesion 20, instead of direct insertion thereof into the lesion 20 on which cauterization is required to be performed may be performed.

Subsequently, Step (a mode selection step) S200 of selecting any one of monopolarity and bipolarity modes may be performed. As an implementation, additionally, a user may select the hybrid mode.

Subsequently, Step (a first switching step) S300 of performing switching in such a manner that one predetermined pair of electrodes, among the first, second, and third electrodes 1000, 2000, and 3000 operates may be performed.

Specifically, when selecting the monopolarity mode, switching is performed in such a manner that the main RF electric power and the sub-RF electric power are supplied to the one predetermined pair of electrodes, respectively. Furthermore, when selecting the bipolarity mode, switching is performed in such a manner that one of the following: the main RF electric power; and the sub-RF electric power is supplied to one of the one predetermined pair of electrodes and that one of the main grounding and sub-grounding lines 140 and 240 is connected to the other one. In addition, when the hybrid mode is selected, switching is performed in such a manner that one of the following: the main RF electric power; and the sub-RF electric power is supplied to one of the one predetermined pair of electrodes and in such a manner that the other one of the one predetermined pair of electrodes and the main ground pad 720 are simultaneously connected to the main grounding line 140, or that the other one of the one predetermined pair of electrodes and the sub-ground pad 740 are simultaneously connected to the sub-grounding line 240. In the present embodiment, Step of switching the first, second, third, and fourth switching units 300, 400, 500, and 600 in such a manner that the first electrode 1000 and the second electrode 2000 operate, when selecting each mode is the same as described above.

Subsequently, Step (a monitoring step) S400 of monitoring voltage and current on the main amplification unit 100 side while the main amplification unit 100 outputs the main RF electric power and monitoring voltage and current on the sub-amplification unit 200 side while the sub-amplification unit 200 outputs the sub-RF electric power may be performed.

In addition, Step (an impedance computation and comparison step) S500 of continuously computing the impedance of the lesion 20 using the monitored voltage and current during the radiofrequency ablation and comparing the computed impedance with the preset reference value may be performed.

Accordingly, Step (a second switching step) S600 of performing, by the control unit 900, switching in such a manner that one pair of electrodes that operate in the monopolarity mode, the bipolarity mode, or the hybrid mode is switched to another pair of electrodes may be performed in the case where the predetermined time elapses or where the computed impedance exceeds the preset reference value. A process in which the pair of the first and second electrodes 1000 and 2000 that operate in each mode is switched to the pair of the second and third electrodes 2000 and 3000 is the same as described above.

Subsequently, the monitoring step S400 and the impedance computation and comparison step S500 are performed. Step (a third switching step) S700 of performing, by the control unit 900, switching in such a manner that one of electrodes that operate in the monopolarity mode, the bipolarity mode, and the hybrid mode is switched to another pair of electrodes may be performed in the case where the predetermined time elapses or the computed impedance exceeds the preset reference value. A process in which the pair of the second and third electrodes 2000 and 3000 that operate in each mode is switched to the pair of the third and first electrodes 3000 and 1000 is the same as described above.

Subsequently, the monitoring step S400 and the impedance computation and comparison step S500 are re-performed. The switching step may be performed in such a manner that the radiofrequency ablation is finished or that the pair of the third and first electrodes 3000 and 1000 that operate in each mode is switched back to the pair of the first and second electrodes 1000 and 2000 may be performed in the case where the predetermined time elapses or where the computed impedance exceeds the preset reference value. Alternatively, the mode may be again selected.

In addition, of course, the mode may be finished or may be switched at any time according to a user's selection.

The present disclosure is not limited to the specific embodiments described above and the description. It would be apparent to a person of ordinary skill in the art to which the present disclosure pertains that various modification embodiments are possible without departing the nature and gist of the present disclosure that is defined in the claims. Such modification embodiments should fall within the scope of the present disclosure.

## Claims

1. A radiofrequency ablation system comprising:
a main amplifier configured to amplify electric power supplied to the main amplifier, the main amplifier being configured to provide main radiofrequency (RF) electric power resulting from amplification by the main amplifier;
a sub-amplifier configured to amplify electric power supplied to the sub-amplifier, the sub-amplifier being configured to provide sub-RF electric power resulting from amplification by the sub-amplifier;
a first switching circuit configured to transfer the main RF electric power provided by the main amplifier to any one of a plurality of electrodes including at least three electrodes;
a second switching circuit configured to transfer the sub-RF electric power provided by the sub-amplifier to any one of the plurality of electrodes;
a third switching circuit configured to connect a main ground pad or any one of the plurality of electrodes to a main grounding line; and
a fourth switching circuit configured to connect a sub-ground pad or any one of the plurality of electrodes to a sub-grounding line,
wherein the radiofrequency ablation system is configured to selectively execute a monopolarity mode or a bipolarity mode according to operations of the first, second, third, and fourth switching circuits,
wherein, in the monopolarity mode, the radiofrequency ablation system is configured to supply the main RF electric power to one of one predetermined pair of electrodes, among the plurality of electrodes, and the sub-RF electric power to the other of the one predetermined pair of electrodes, and
wherein, in the bipolarity mode, the radiofrequency ablation system is configured to supply either the main RF electric power or the sub-RF electric power to one of the one predetermined pair of electrodes, the radiofrequency ablation system being configured to connect either the main grounding line or the sub-grounding line to the other of the one predetermined pair of electrodes.

2. The radiofrequency ablation system of claim 1, wherein the plurality of electrodes is configured to be inserted into a surrounding tissue to surround a lesion, without direct insertion into the lesion.

3. The radiofrequency ablation system of claim 1 or 2, wherein, in the monopolarity mode, the main ground pad is configured to be connected to the main grounding line, and the sub-ground pad is configured to be connected to the sub-grounding line.

4. The radiofrequency ablation system of one of claims 1 to 3, wherein, in the bipolarity mode, when the main RF electric power is supplied to one of the one predetermined pair of electrodes, the main grounding line is configured to be connected to the other of the one predetermined pair of electrodes, and, when the sub-RF electric power is supplied to one of the one predetermined pair of electrodes, the sub-grounding line is configured to be connected to the other of the one predetermined pair of electrodes.

5. The radiofrequency ablation system of one of claims 1 to 4, wherein the one predetermined pair of electrodes that operate in the monopolarity mode or the bipolarity mode is configured to be switched to another pair of electrodes when a predetermined time elapses or according to an impedance value of a lesion.

6. The radiofrequency ablation system of one of claims 1 to 5, further comprising:
a main board sensor configured to monitor voltage and current on a main amplifier side while the main amplifier outputs the main RF electric power; and
a sub-sensor board configured to monitor voltage and current on a sub-amplifier side while the sub-amplifier outputs the sub-RF electric power.

7. The radiofrequency ablation system of claim 6, further comprising:
a controller configured to continuously compute impedance of a lesion using the voltage and the current that are monitored by the main board sensor and the voltage and the current that are monitored by the sub-sensor board, during radiofrequency ablation, the controller being configured to compare the impedance with a preset reference value.

8. The radiofrequency ablation system of claim 7, wherein, in a case where a predetermined time elapses or in a case where the impedance exceeds the preset reference value, the controller is configured to control the first, second, third, and fourth switching circuits to switch the one predetermined pair of electrodes that operate in the monopolarity mode or the bipolarity mode to another pair of electrodes.

9. The radiofrequency ablation system of claim 5,
wherein the radiofrequency ablation system is configured to selectively execute one of the monopolarity mode, the bipolarity mode, and a hybrid mode, and wherein, in the hybrid mode, the radiofrequency ablation system is configured to supply either the main RF electric power or the sub-RF electric power to one of the one predetermined pair of electrodes, and the other of the one predetermined pair of electrodes and the main ground pad are configured to be simultaneously connected to the main grounding line, or the other of the one predetermined pair of electrodes and the sub-ground pad are configured to be simultaneously connected to the sub-grounding line.

10. The radiofrequency ablation system of claim 9, wherein the one predetermined pair of electrodes that operate in the hybrid mode is configured to be switched to another pair of electrodes when the predetermined time elapses or according to the impedance value of the lesion.

11. The radiofrequency ablation system of one of claims 1 to 10,
wherein, in a case where the plurality of electrodes includes first, second, and third electrodes, the first switching circuit comprises:
a first switch configured to transfer the main RF electric power to the first electrode;
a second switch configured to transfer the main RF electric power to the second electrode; and
a third switch configured to transfer the main RF electric power to the third electrode, and
wherein the second switching circuit comprises:
an eighth switch configured to transfer the sub-RF electric power to the first electrode;
a ninth switch configured to transfer the sub-RF electric power to the second electrode; and
a tenth switch configured to transfer the sub-RF electric power to the third electrode.

12. The radiofrequency ablation system of claim 11,
wherein, the third switching circuit comprises:
a fourth switch configured to connect the main ground pad to the main grounding line;
a fifth switch configured to connect the first electrode to the main grounding line;
a sixth switch configured to connect the second electrode to the main grounding line; and
a seventh switch configured to connect the third electrode to the main grounding line, and
wherein the fourth switching circuit comprises:
an eleventh switch configured to connect the sub-ground pad to the sub-grounding line;
a twelfth switch configured to connect the first electrode to the sub-grounding line;
a thirteenth switch configured to connect the second electrode to the sub-grounding line; and
a fourteenth switch configured to connect the third electrode to the sub-grounding line.

13. A method of controlling radiofrequency ablation system that uses a plurality of electrodes having at least three electrodes, the method comprising:
selecting either monopolarity mode or bipolarity mode;
performing switching
in a manner that main radiofrequency (RF) electric power is supplied to one of one predetermined pair of electrodes, among the plurality of electrodes, and sub-RF electric power is supplied to the other of the one predetermined pair of electrodes, when selecting the monopolarity mode, and
in a manner that either the main RF electric power or the sub-RF electric power is supplied to one of the one predetermined pair of electrodes, and either main grounding line or a sub-grounding line is connected to the other of the predetermined pair of electrodes, when selecting the bipolarity mode;
monitoring voltage and current on a main amplifier side while a main amplifier outputs the main RF electric power, and monitoring voltage and current on a sub-amplifier side while a sub-amplifier outputs the sub-RF electric power;
computing continuously impedance of a lesion using the voltage and the current on the main amplifier side and the voltage and the current on the sub-amplifier side during radiofrequency ablation and comparing the impedance with a preset reference value; and
performing another switching, wherein the one predetermined pair of electrodes that operate in the monopolarity mode or the bipolarity mode is switched to another pair of electrodes, in a case where a predetermined time elapses or where the impedance exceeds the preset reference value.

14. The method of claim 13, further comprising:
inserting the plurality of electrodes in a surrounding tissue to surround a lesion, without direct insertion into the lesion on which cauterization is to be performed.

15. The method of claim 13 or 14,
wherein the selecting is performed by selecting one of the monopolarity mode, the bipolarity mode, and a hybrid mode,
wherein, when the hybrid mode is selected, switching in the hybrid mode is performed
in a manner that either the main RF electric power or the sub-RF electric power is supplied to one of the one predetermined pair of electrodes and
in a manner that the other of the one predetermined pair of electrodes and a main ground pad are simultaneously connected to the main grounding line by the switching in the hybrid mode, or that the other of the one predetermined pair of electrodes and a sub-ground pad are simultaneously connected to the sub-grounding line by the switching in the hybrid mode, and
wherein another switching in the hybrid mode is performed, wherein the one predetermined pair of electrodes that operate in the hybrid mode is switched to another pair of electrodes, in the case where the predetermined time elapses or where the impedance exceeds the preset reference value.
